# EUROPÄISCHE PATENTSCHRIFT

(11) **EP 0 949 237 B1**
(45) Veröffentlichungstag und Bekanntmachung des Hinweises auf die Patenterteilung: **18.02.2004**
(21) Anmeldenummer: 99106506.1
(22) Anmeldetag: 30.03.1999
(51) Int. Cl.: C07C 47/21

(54) **Verfahren zur Herstellung von Dihydrocitral**
Process for the preparation of dihydrocitral
Procédé pour la préparation de dihydrocitral

(30) Priorität: 07.04.1998 EP 98106299
(43) Veröffentlichungstag der Anmeldung: 13.10.1999
(73) Patentinhaber: F. HOFFMANN-LA ROCHE AG, 4070 Basel (CH)
(72) Erfinder: Bonrath, Werner, 79115 Freiburg (DE)

(56) Entgegenhaltungen:
- EP-A- 0 240 431
- GB-A- 1 204 754
- LORBER C Y ET AL: "Cis-dioxomolybdenum(VI) Complexes as New Catalysts for the Meyer -Schuster Rearrangement" TETRAHEDRON LETTERS, Bd. 37, Nr. 6, 5. Februar 1996 (1996-02-05), Seite 853-856 XP004030279 ISSN: 0040-4039
- ERMAN, M.B. ET AL: "The rearrangement of tertiary propargyl alcohols to alpha,beta-unsaturated aldehydes in the presence of polymeric organosilyl vanadetes" TETRAHEDRON LETT., Nr. 34, 1976, Seiten 2981-2984, XP002111028

## Beschreibung

Die vorliegende Erfindung betrifft ein neues Verfahren zur Herstellung von Dihydrocitral durch eine besondere katalysierte Umlagerung von Dihydrodehydrolinalool. Der α,β-ungesättigte Aldehyd Dihydrocitral (E/Z-3,7-Dimethyl-2-octen-1-al) ist ein nützliches Zwischenprodukt.

α,β-Ungesättigte Carbonylverbindungen sind im allgemeinen wichtige Zwischenprodukte für die Herstellung von Geruchsstoffen, Vitaminen und Carotinoiden [siehe beispielsweise Chem. Ztg. 97, 23-28 (1973) und Kap. VI ("Total Syntheses") in "Carotenoids", Ed. Otto Isler, Birkhäuser Verlag Basel und Stuttgart, 1971]. Ihre Herstellung durch säurekatalysierte Umlagerung von α-Alkinolen wurde bereits in den zwanziger Jahren von K. H. Meyer und K. Schuster [Ber. deutsch. Chem. Ges. 55, 819-823 (1922)] und H. Rupe und E. Kambli [Helv. Chim. Acta 9, 672 (1926)] beschrieben; die Isomerisierung sekundärer oder tertiärer α-Alkinole zu α,β-ungesättigten Carbonylverbindungen ist auch allgemein als Meyer-Schuster- bzw. Rupe-Kambli-Umlagerung bekannt geworden. Handelt es sich um die Umlagerung einer Carbonylverbindung mit einer endständigen Alkinylgruppe, werden Aldehyde erhalten, andernfalls sind Ketone die Umlagerungsprodukte: 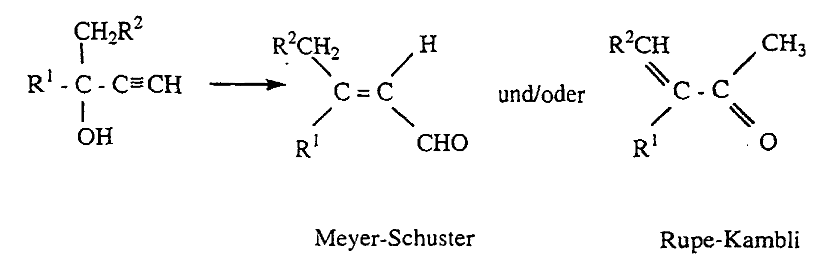 worin R¹ und R² jeweils Wasserstoff oder einen aliphatischen oder aromatischen Rest bedeuten.

Die durch Silber- oder Kupferionen katalysierte Umlagerung von Dihydrodehydrolinalylacetat liefert nach G. Saucy et al. [Helv. Chim. Acta 42, 1945-1955 (1959)] in Abhängigkeit von den Reaktionsbedingungen ein Gemisch aus "Allenacetat" (1-Acetoxy-3,7-dimethyl-octa-1,2-dien) und "Diacetat" (1,1-Diacetoxy-3,7-dimethyl-2-octen), das sich zu Dihydrocitral hydrolysieren lässt: 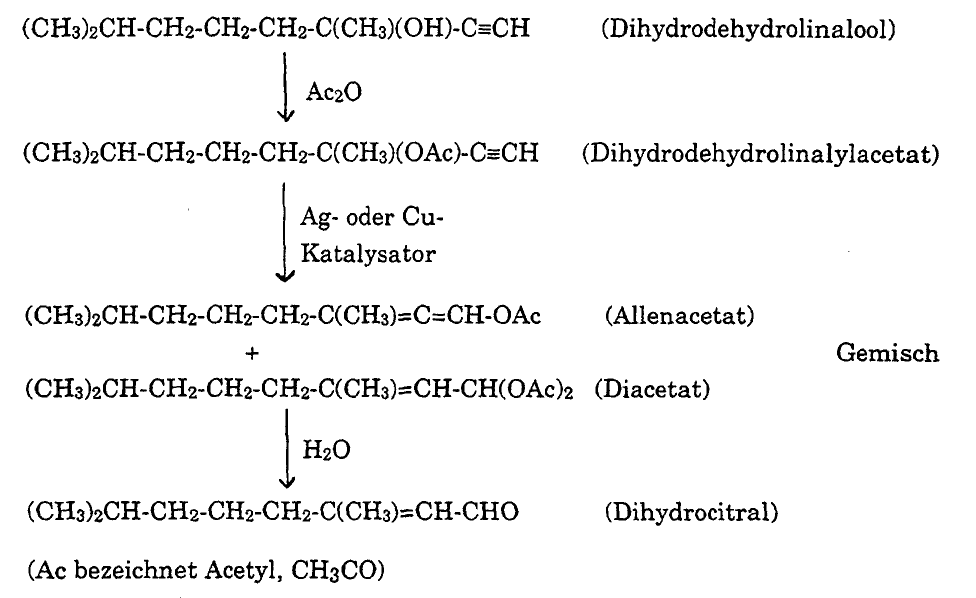 Diese Umlagerung von Dihydrodehydrolinalylacetat ist als Saucy-Marbet-Umlagerung bekannt.

Gegenüber der von C.C. Price und J.A. Pappalardo in J.A.C.S. 72, 2613 (1950) beschriebenen Synthese von Dihydrocitral ausgehend von Isoheptanoylchlorid weist die Saucy-Marbet-Umlagerung die Vorteile einer höheren Ausbeute, nämlich 80% gegenüber 20%, und der Vermeidung stark tränenreizender Zwischenprodukte auf. Nachteilig ist allerdings die Verwendung eines Silber- oder Kupfer-enthaltenden Katalysators.

Weitere bekannte Methoden zur Herstellung von Dihydrocitral sind die bei 350°C in Biphenyl durchgeführte Umlagerung von 3-Methyl-1-(3-methylbutoxy)-buta-1,3-dien [Japanische Patentpublikation 203025/1982)/Chem. Abs. 99, 5873r (1983)] und die in 59%iger Ausbeute verlaufende Oxidation von 3,7-Dimethyl-oct-2-en-1-ol mit Silbercarbonat auf Celite ® [Fetizon's Reagens; B.C.L. Weedon und Mitarbeiter, J. Chem. Soc. Perkin Trans. 1, 1457 (1975)]. Nachteilig sind die hohe Reaktionstemperatur bzw. die Verwendung eines Silber-enthaltenden Katalysators.

Eine interessante Variante der oben erwähnten Meyer-Schuster-Umlagerung wurde kürzlich von C.Y. Lorber und J.A. Osborn in Tetr. Lett. 37, 853-856 (1996) beschrieben; es handelt sich dabei um die Umlagerung von Methylbutinol zu Prenal unter Verwendung eines Molybdän-Katalysators. Hierbei wird in ortho-Dichlorbenzol als Lösungsmittel Methylbutinol in Gegenwart des Katalysatorsystems Molybdänylacetylacetonat, Dibutylsulfoxid und 4-tert. Butylbenzoesäure zu Prenal umgelagert. Obwohl die Ausbeute und Selektivität dieser Umlagerung mit 90% bzw. 99% angegeben sind, wurde das Prenal nicht aus dem Reaktionsgemisch isoliert, sondern die angebliche Ausbeute durch gaschromatographische Analyse des rohen Produktes eruiert. Vermutlich war es schwierig, das Reaktionsgemisch aufzuarbeiten, um Prenal zu isolieren.

L.A. Kheifits und Mitarbeiter fanden, dass Dehydrolinalool bei 170°C in 14 Stunden Reaktionszeit lediglich in 28%iger Ausbeute in Citral und in 12%iger Ausbeute in 2-Hydroxymethyl-1-methyl-3-isopropenylcyclopent-1-en übergeführt werden konnte, wenn als Katalysator ein aus Natriummolybdänat und Diphenyldichlorsilan hergestelltes Molybdän-Polymer für die Umlagerung verwendet wurde [Tetr. Lett. 34, 2981-2984 (1976)].

UK-Patentschrift 1.204.754 beschreibt die Umlagerung von u.a. Dehydrolinalool zu Citral unter Verwendung eines auf einem Uebergangsmetall der Untergruppe Vb, VIb oder VIIb des Periodensystems der Elemente basierenden Katalysators, z.B. einer Molybdänverbindung (siehe insbesondere Beispiele 22-24). In Beispiel 23 handelt es sich bei dem Katalysator vermutlich entweder um Molybdänylacetylacetonat (MoO₂acac₂) selber oder zumindest um ein MoO₂acac₂ enthaltendes Gemisch, dessen Herstellung durch Erhitzen von MoO₃ und Acetylaceton offenbart ist, allerdings ohne chemische Identifizierung des so hergestellte Katalysators. Das Beispiel offenbart weiter die katalysierte Umlagerung von Dehydrolinalool zu Citral in der Abwesenheit eines Lösungsmittels und eines weiteren Katalysators oder sonstigen reaktionsfördernden Stoffes ("Aktivators"). Auch die allgemeine Beschreibung steht nicht im Widerspruch zur Unbestimmtheit betreffend die Anwesenheit oder Abwesenheit eines Lösungsmittels, eines Ko-Katalysators oder eines Aktivators; in der , Patentschrift ist auf jeden Fall weder die Rede von der Verwendung eines Sulfoxids in der Umlagerung, noch die Rede von einem sauren organischen Ko-Katalysator bzw. Aktivator mit einem bestimmten pK-Wert. Ferner gibt Beispiel 23 weder die Ausbeute noch den Umsatzgrad an, sondern bloss den Anteil an Citral im produzierten Gemisch; wohl waren die Ausbeute und der Umsatzgrad eher niedrig. Auch unter Berücksichtigung der weiteren Beispiele, welche die Verwendung von Molybdän enthaltenden Katalysatoren veranschaulichen, und in denen die Ergebnisse (Ausbeute und Umsatzgrad) angegeben sind (Beispiele 22, 24 sowie 35), sind die Ausbeuten und Umsatzgrade im Allgemeinen niedriger als die durch die Verwendung von Vanadium enthaltenden Katalysatoren erzielten Ausbeuten bzw. Umsatzgrade.

Aus den obigen Ausführungen ist ersichtlich, dass die vorbekannten Verfahren zur katalysierten Umlagerung von α-Alkinolen zu α,β-ungesättigten Aldehyden, z.B. Dehydrolinalool zu Citral, gewichtige Nachteile aufweisen, was auch vermutlich für die analoge Umlagerung von Dihydrodehydrolinalool zu Dihydrocitral zutreffen würde.

Der vorliegenden Erfindung liegt nun die Aufgabe zugrunde, ein Verfahren zur Umlagerung von Dihydrodehydrolinalool zu Dihydrocitral unter Einwirkung eines Katalysators bereitzustellen, das die Nachteile der vorbekannten Verfahren nicht oder zumindest viel weniger aufweist. Diese Aufgabe wird überraschend gut gelöst, indem man nicht nur die bekannten Molybdänverbindung Molybdänylacetylacetonat [auch als Dioxomolybdän(VI)-acetylacetonat bekannt] oder ein Molybdänylhalogenid als Katalysator verwendet, sondern diese Verbindung als Bestandteil eines bestimmten Katalysatorsystems einsetzt und ansonsten die Umlagerung unter gewissen Reaktionsbedingungen durchführt.

Bei dem erfindungsgemässen Verfahren handelt es sich um ein Verfahren zur Herstellung von Dihydrocitral durch katalysierte Umlagerung von Dihydrodehydrolinalool zu Dihydrocitral, das dadurch gekennzeichnet ist, dass man die Umlagerung in Gegenwart einer Molybdänverbindung der allgemeinen Formel

MoO₂X₂ I

worin X einen Acetylacetonat- oder Halogenidion bedeutet
, und eines Dialkyl- oder Diarylsulfoxids als Katalysatorsystem, in Gegenwart einer organischen Säure mit einem pK-Wert im Bereich von 4,0 bis 6,5 sowie in einem aprotischen organischen Lösungsmittel durchführt.

Die Molybdänverbindung der Formel I, d.h. Molybdänylacetylacetonat (konventionell als MoO₂acac₂ bezeichnet) oder ein Molybdänylhalogenid der Formel MoO₂(Hal)₂ [X = Hal], worin Hal Chlor oder Brom bedeutet, ist jeweils eine im Handel leicht erhältliche bekannte Verbindung. Das Molybdänylhalogenid ist vorzugsweise Molybdänylchlorid, MoO₂Cl₂. Die bevorzugte Molybdänverbindung der Formel I ist allerdings Molybdänylacetylacetonat.

Bei dem ebenfalls im Katalysatorsystem vorhandenen Dialkyl- oder Diarylsulfoxid handelt es sich insbesondere um ein Dialkylsulfoxid, dessen Alkylgruppen jeweils geradkettig oder verzweigt sind und bis zu 8 Kohlenstoffatome enthalten, bzw. um ein Diarylsulfoxid, dessen Arylgruppen jeweils gegebenenfalls substituierte Phenylgruppen sind. Im letzteren Fall können die allfällig vorhandenen Substituenten insbesondere C₁₋₄-Alkylgruppen sein, wobei die Phenylgruppe jeweils einfach oder mehrfach alkylsubstituiert ist. Beispiele beider Typen von Sulfoxiden sind Dimethylsulfoxid und Dibutylsulfoxid bzw. Diphenylsulfoxid und Di(p-tolyl)sulfoxid. Vorzugsweise wird als Sulfoxid das Dimethylsulfoxid verwendet.

Als organische Säuren mit einem pK-Wert im Bereich von 4,0 bis 6,5 kommen u.a. in Frage gegebenenfalls halogenierte, gesättigte und ungesättigte aliphatische Carbonsäuren, z.B. Essigsäure (pK-Wert 4,74), Propionsäure (4,87), Chlorpropionsäure (3,98) und Pivalinsäure (5,01) bzw. Acrylsäure (4,25); Alkandicarbonsäuren, z.B. Adipinsäure (4,40); arylsubstituierte Alkancarbonsäuren, z.B. Phenylessigsäure (4,25); sowie aromatische Carbonsäuren, z.B. Benzoesäure (4,19) und 4-tert.Butyl-benzoesäure (6,50). Vorzugsweise verwendet man eine organische Säure mit einem pK-Wert im Bereich von 4,25 bis 6,5, insbesondere Acrylsäure mit dem pK-Wert 4,25.

Als Lösungsmittel können im Rahmen der vorliegenden Erfindung im allgemeinen apolare aprotische organische Lösungsmittel, insbesondere aliphatische, cyclische und aromatische Kohlenwasserstoffe, wie beispielsweise C₇₋₁₀-Alkane, C₅₋₇-Cycloalkane, Benzen, Toluen und Naphthalen sowie Gemische solcher Lösungsmittel untereinander, z.B. Paraffinöl (Gemisch gesättigter aliphatischer Kohlenwasserstoffe), und polare aprotische organische Lösungsmittel, insbesondere aliphatische und cyclische Ester mit bis zu etwa 6 Kohlenstoffatomen, wie beispielsweise Ethylacetat und Butylacetat bzw. Ethylencarbonat, Propylencarbonat und Butyrolacton, verwenden werden. Toluen stellt ein besonders bevorzugtes Lösungsmittel dar.

Die Umlagerung erfolgt zweckmässigerweise bei Temperaturen im Bereich von 80°C bis 140°C, vorzugsweise bei Temperaturen von 90°C bis 120°C.

Die Menge Molybdänverbindung der Formel I beträgt zweckmässigerweise etwa 0,1 - 8 Mol-% bezogen auf die Menge eingesetzten Dihydrodehydrolinalools (Edukt). Diese Menge beträgt vorzugsweise etwa 1-7 Mol-%, ganz bevorzugt etwa 3-5 Mol-%.

Darüber hinaus beträgt das Gewichtsverhältnis Dialkyl- oder Diarylsulfoxid zu Edukt zweckmässigerweise 0,2:1 bis 1:1; das Gewichtsverhältnis Säure zu Edukt zweckmässigerweise 0,02:1 bis 0,1:1, vorzugsweise 0,04:1 bis 0,07:1, besonders bevorzugt 0,05:1; und das Gewichtsverhältnis Lösungsmittel zu Edukt zweckmässigerweise 5:1 bis 15:1, vorzugsweise 7:1 bis 10:1.

Das erfindungsgemässe Verfahren kann arbeitstechnisch sehr einfach durchgeführt werden, indem man das Edukt, das Katalysatorsystem (Molybdänverbindung der Formel I sowie Dialkyl- oder Diarylsulfoxid) und die organische Säure zum Lösungsmittel gibt und das Reaktionsgemisch, welches normalerweise wegen der unterschiedlichen Löslichkeiten der Reaktionsteilnehmer aus einer Suspension besteht, auf die Reaktionstemperatur erhitzt. Die Reihenfolge der Zugabe ist nicht kritisch, so dass beispielsweise die Säure oder das Sulfoxid zuletzt zugegeben werden kann. Zur Reaktionskontrolle können Proben entnommen und nach bekannten Methoden, z.B. Dünnschichtchromatographie oder Gaschromatographie, analysiert werden. Nach beendeter Reaktion, wobei die Reaktionsdauer normalerweise bis zu 20 Stunden, vorzugsweise bis zu 7 Stunden, beträgt, kann die Aufarbeitung durch gängige Verfahren der organischen Chemie erfolgen. Typischerweise wird das Gemisch filtriert und das Produkt Dihydrocitral aus dem Filtrat durch Eindampfen isoliert. Zur Reinigung des Produktes kann die Rohware beispielsweise destilliert werden.

Das erfindungsgemässe Verfahren wird durch die nachfolgenden Beispiele veranschaulicht:

### Beispiel 1

### Verschiedene Versuche der Umlagerung unter gleichen Bedingungen

In einem mit Thermometer, Rührer und Rückflusskühler versehenen 100 ml-Sulfierkolben wurden 6,24 g (39,62 mmol) Dihydrodehydrolinalool (nachfolgend "DDLL"), 2,31 g (29,67 mmol) Dimethylsulfoxid (nachfolgend "DMSO"), 0,65 g (1,98 mmol) Molybdänylacetylacetonat (nachfolgend "MoO₂acac₂") und 1,05 g (14,58 mmol) Acrylsäure in 50 ml Toluen vorgelegt. Anschliessend wurde auf 100°C erwärmt. Hierbei entstand aus der zunächst rotbraunen Suspension eine grünbraune Lösung. Zur Reaktionskontrolle wurden Proben entnommen und mittels Dünnschichtchromatographie (DC) oder Gaschromatographie (GC) analysiert. Nach beendeter Reaktion (17 Stunden Reaktionszeit) wurde aufgearbeitet, indem auf Raumtemperatur gekühlt, über 10 g Kieselgel filtriert, das Kieselgel mit 100 ml Toluen nachgewaschen und schliesslich bei 40°C und 70 mbar (7 kPa) Druck eingeengt wurde. Auf diese Weise erhielt man 7,61 g Rohware, die gemäss Gehaltsbestimmung durch GC mit internem Standard zu 4,69% aus unreagiertem DDLL (0,37 g, 5,93 Mol.% der eingesetzten 6,24 g) und zu 61,05% aus gewünschtem Dihydrocitral (4,78 g, 76,61 Mol.%ige Ausbeute) bestand. Dies ergibt ein prozentuelles Verhältnis von Dihydrocitral (nachfolgend "DHC") zu DDLL von 81,43% [(g DHC ö g umgesetztes DDLL, 5,87 g) x 100].

Der obige Versuch (Nr.1) wurde noch dreimal wiederholt, und zwar mit unveränderten Mengen und Reaktions- und Aufarbeitungsbedingungen. Es wurden die in der nachfolgenden Tabelle zusammengefassten Ergebnisse erhalten:

**Tabelle**

| Versuch-Nr. | Umgesetztes DDLL | Ausbeute in Mol.% | | Ausbeute in g | | Proz. Verhältnis DHC:DDLL |
|---|---|---|---|---|---|---|
| | | DDLL | DHC | DDLL | DHC | |
| 2 | 5,87 g | 11,54 | 74,19 | 0,72 | 4,63 | 83,88% |
| 3 | 5,00 g | 19,71 | 67,26 | 1,23 | 4,20 | 84,00% |
| 4 | 5,44 g | 12,82 | 71,28 | 0,80 | 4,45 | 81,80% |

### Beispiel 2

### Umlagerung unter Verwendung von Butylacetat als Lösungsmittel

Wie in Beispiel 1 beschrieben wurde eine Suspension von 6,24 g (39,62 mmol) DDLL, 2,31 g (29,67 mmol) DMSO, 0,65 g (1,98 mmol) MoO₂acac₂ und 1,05 g (14,58 mmol) Acrylsäure in 50 ml Butylacetat auf 100°C erwärmt. Hierbei entstand aus der zunächst rotbraunen Suspension eine grüne Lösung. Nach 17 Stunden Reaktionszeit wurde auf Raumtemperatur gekühlt und die Reaktionslösung über 10 g Kieselgel filtriert. Das Kieselgel wurde mit 100 ml Toluen nachgewaschen und das Lösungsmittel bei 40°C und 70 mbar (7 kPa) abgedampft. Auf diese Weise erhielt man 10,2 g Rohware, die gemäss Gehaltsbestimmung durch GC mit internem Standard zu 41,12% aus DHC bestand und kein unreagiertes DDLL aufwies. Der Gehalt DHC betrug 4,19 g (67,22%ige Ausbeute).

## Patentansprüche

1. Verfahren zur Herstellung von Dihydrocitral durch katalysierte Umlagerung von Dihydrodehydrolinalool zu Dihydrocitral, **dadurch gekennzeichnet, dass** man die Umlagerung in Gegenwart einer Molybdänverbindung der allgemeinen Formel
MoO₂X₂ I
worin X einen Acetylacetonat- oder Halogenidion bedeutet,
und eines Dialkyl- oder Diarylsulfoxids als Katalysatorsystem, in Gegenwart einer organischen Säure mit einem pK-Wert im Bereich von 4,0 bis 6,5 sowie in einem aprotischen organischen Lösungsmittel durchführt.

2. Verfahren nach Anspruch 1, **dadurch gekennzeichnet, dass** die Molybdänverbindung der Formel I Molybdänylacetylacetonat oder Molybdänylchlorid, vorzugsweise Molybdänylacetylacetonat, ist.

3. Verfahren nach Anspruch 1 oder 2, **dadurch gekennzeichnet, dass** das Dialkyl- oder Diarylsulfoxid Dimethylsulfoxid oder Dibutylsulfoxid bzw. Diphenylsulfoxid oder Di(p-tolyl)sulfoxid, vorzugsweise Dimethylsulfoxid, ist.

4. Verfahren nach einem der Ansprüche 1 bis 3, **dadurch gekennzeichnet, dass** als organische Säure eine gegebenenfalls halogenierte, gesättigte oder ungesättigte aliphatische Carbonsäure, eine Alkandicarbonsäure, eine arylsubstituierte Alkancarbonsäure oder eine aromatische Carbonsäure verwendet wird.

5. Verfahren nach Anspruch 4, **dadurch gekennzeichnet, dass** die organische Säure Essigsäure, Propionsäure, Chlorpropionsäure, Pivalinsäure, Acrylsäure, Adipinsäure, Phenylessigsäure, Benzoesäure oder 4-tert.Butylbenzoesäure, vorzugsweise Acrylsäure, ist.

6. Verfahren nach einem der Ansprüche 1 bis 5, **dadurch gekennzeichnet, dass** als Lösungsmittel ein aliphatischer Kohlenwasserstoff, ein cyclischer Kohlenwasserstoff, ein aromatischer Kohlenwasserstoff, ein Gemisch solcher Lösungsmittel untereinander oder ein aliphatischer oder cyclischer Ester mit bis zu etwa 6 Kohlenstoffatomen verwendet wird.

7. Verfahren nach Anspruch 6, **dadurch gekennzeichnet, dass** das Lösungsmittel ein C₇₋₁₀-Alkan, ein C₅₋₇-Cycloalkan, Benzen, Toluen, Naphthalen, Paraffinöl, Ethylacetat, Butylacetat, Ethylencarbonat, Propylencarbonat oder Butyrolacton, vorzugsweise Toluen, ist.

8. Verfahren nach einem der Ansprüche 1 bis 7, **dadurch gekennzeichnet, dass** die Umlagerung bei Temperaturen im Bereich von 80°C bis 140°C, vorzugsweise bei Temperaturen von 90°C bis 120°C, erfolgt.

9. Verfahren nach einem der Ansprüche 1 bis 8, **dadurch gekennzeichnet, dass** die Menge Molybdänverbindung der Formel I 0,1 - 8 Mol-% bezogen auf die Menge eingesetzten Dihydrodehydrolinalools, vorzugsweise 1-7 Mol-%, ganz bevorzugt 3-5 Mol-%, beträgt.

10. Verfahren nach einem der Ansprüche 1 bis 9, **dadurch gekennzeichnet, dass** das Gewichtsverhältnis Dialkyl- oder Diarylsulfoxid zu Dihydrodehydrolinalool (Edukt) zweckmässigerweise 0,2:1 bis 1:1; das Gewichtsverhältnis Säure zu Edukt zweckmässigerweise 0,02:1 bis 0,1:1, vorzugsweise 0,04:1 bis 0,07:1, besonders bevorzugt 0,05:1; und das Gewichtsverhältnis Lösungsmittel zu Edukt 5:1 bis 15:1, vorzugsweise 7:1 bis 10:1, beträgt.

## Claims

1. A process for the manufacture of dihydrocitral by the catalytic rearrangement of dihydrodehydrolinalool to dihydrocitral, which process comprises carrying out the rearrangement in the presence of a molybdenum compound of the general formula
MoO₂X₂ I
wherein X signifies an acetylacetonate or halide ion,
and a dialkyl or diaryl sulphoxide as the catalyst system, in the presence of an organic acid having a pK value in the range of 4.0 to 6.5 as well as in an aprotic organic solvent.

2. A process according to claim 1, wherein the molybdenum compound of formula I is molybdenyl acetylacetonate or molybdenyl chloride, preferably molybdenyl acetylacetonate.

3. A process according to claim 1 or claim 2, wherein the dialkyl or diaryl sulphoxide is dimethyl sulphoxide or dibutyl sulphoxide or, respectively, diphenyl sulphoxide or di(p-tolyl) sulphoxide, preferably dimethyl sulphoxide.

4. A process according to any one of claims 1 to 3, wherein an optionally halogenated, saturated or unsaturated aliphatic carboxylic acid, an alkanedicarboxylic acid, an aryl-substituted alkanecarboxylic acid or an aromatic carboxylic acid is used as the organic acid.

5. A process according to claim 4, wherein the organic acid is acetic acid, propionic acid, chloropropionic acid, pivalic acid, stearic acid, acrylic acid, adipic acid, phenylacetic acid, benzoic acid or 4-tert.butyl-benzoic acid, preferably acrylic acid.

6. A process according to any one of claims 1 to 5, wherein an aliphatic hydrocarbon, a cyclic hydrocarbon or an aromatic hydrocarbon, a mixture of such solvents with one another or an aliphatic or cyclic ester with up to 6 carbon atoms is used as the solvent.

7. A process according to claim 6, wherein the solvent is a C₇₋₁₀-alkane, a C₅₋₇-cycloalkane, benzene, toluene, naphthalene, paraffin oil, ethyl acetate, butyl acetate, ethylene carbonate, propylene carbonate or butyronitrile, preferably toluene.

8. A process according to any one of claims 1 to 7, wherein the rearrangement is effected at temperatures in the range of 80°C to 140°C, preferably at temperatures of 90°C to 120°C.

9. A process according to any one of claims 1 to 8, wherein the amount of molybdenum compound of formula I is 0.1-8 mol% based on the amount of dihydrodehydrolinalool employed, preferably 1-7 mol%, particularly 3-5 mol%.

10. A process according to any one of claims 1 to 9, wherein the weight ratio of dialkyl or diaryl sulphoxide to dihydrodehydrolinalool (educt) is conveniently 0.2:1 to 1:1; the weight ratio of acid to educt is conveniently 0.02:1 to 0.1:1, preferably 0.04:1 to 0.07:1, particularly 0.05:1; and the weight ratio of solvent to educt is 5:1 to 15:1, preferably 7:1 to 10:1.

## Revendications

1. Procédé de fabrication de dihydrocitral par réarrangement catalysé de dihydrodéhydrolinalool en dihydrocitral, **caractérisé en ce que** le réarrangement a lieu en présence d'un composé de molybdène de formule générale
MoO₂X₂ I
dans laquelle X est un ion acétylacétonate ou halogénide,
et d'un dialkyle- ou diarylesulfoxyde comme système catalysateur, en présence d'un acide organique avec une valeur de pK comprise dans la plage allant de 4,0 à 6,5 et dans un solvant organique aprotique.

2. Procédé selon la revendication 1, **caractérisé en ce que** le composé de molybdène de formule I est de l'acétylaçétonate de molybdènyle ou du chlorure de molybdènyle de préférence de l'acétylacétonate de molybdènyle

3. Procédé selon la revendication 1 ou 2, **caractérisé en ce que** le dialkyl- ou diarylsulfoxyde est du diméthylsulfoxyde ou du dibutylsulfoxyde ou du diphénylsulfoxyde ou du di(p-tolyl)sulfoxyde, de préférence du diméthylsulfoxyde.

4. Procédé selon l'une des revendications 1 à 3, **caractérisé en ce que** comme acide organique on utilise un acide carboxylique aliphatique éventuellement halogéné, saturé ou insaturé, un acide alcanedicarboxylique, un acide alcanecarboxylique substitué par aryle ou un acide carboxylique aromatique.

5. Procédé selon la revendication 4, **caractérisé en ce que** l'acide organique est de l'acide acétique, de l'acide propionique, de l'acide chlorpropionique, de l'acide pivalique, de l'acide acrylique, de l'acide adipique, de l'acide phénylacétique, de l'acide benzoïque ou de l'acide 4-tert-butylbenzoïque, de préférence de l'acide acrylique.

6. Procédé selon une des revendications 1 à 5, **caractérisé en ce qu'**on utilise comme solvant un hydrocarbure aliphatique, un hydrocarbure cyclique, un hydrocarbure aromatique, un mélange de ces solvants ou un ester aliphatique ou cyclique comptant jusqu'à 6 atomes de carbone.

7. Procédé selon la revendication 6, **caractérisé en ce que** le solvant est un alcane en C₇₋₁₀, un cycloalcane en C₅₋₇, le benzène, le toluène, le naphtalène, l'huile de paraffine, l'acétate d'éthyle, l'acétate de butyle, le carbonate d'éthylène, le carbonate de propylène ou le butyrolactone, de préférence le toluène.

8. Procédé selon les revendications 1 à 7, **caractérisé en ce que** le réarrangement a lieu à des températures dans la plage allant de 80°C à 140°C, de préférence à des températures de 90°C à 120°C.

9. Procédé selon l'une des revendications 1 à 8, **caractérisé en ce que** la quantité de composé de molybdène de formule I s'élève de 0,1 à 8 % molaires de la quantité de dihydrodéhydrolinalool utilisée, de préférence de 1 à 7 % molaires, de manière préférée entre toutes de 3 à 5 % molaires.

10. Procédé selon l'une des revendications 1 à 9, **caractérisé en ce que** le rapport de poids de dialkylsulfoxyde ou de diarylsulfoxyde sur le dihydrodéhydrolinalool (matière de départ) s'élève de manière appropriée de 0,2 : 1 à 1 : 1 ; le rapport de poids de l'acide sur la matière de départ s'élève de manière appropriée de 0,02 : 1 à 0,1 : 1, de préférence de 0,04 : 1 à 0,07 : 1, de manière particulièrement préférée de 0,05 : 1 ; et le rapport de poids du solvant sur la matière de départ s'élève de 5 : 1 à 15 : 1, de préférence de 7 : 1 à 10 : 1.
